(19) Europäisches Patentamt
European Patent Office
Office européen des brevets

(11) Publication number: **0 056 679**
**B1**

(12) # EUROPEAN PATENT SPECIFICATION

(45) Date of publication of patent specification: **17.04.85**

(51) Int. Cl.$^4$: **C 07 C 29/32,** C 07 C 31/08

(21) Application number: **82300010.4**

(22) Date of filing: **04.01.82**

(54) **Ethanol synthesis by homologation of methanol.**

(30) Priority: **08.01.81 US 223514**
**08.01.81 US 224199**

(43) Date of publication of application:
**28.07.82 Bulletin 82/30**

(45) Publication of the grant of the patent:
**17.04.85 Bulletin 85/16**

(84) Designated Contracting States:
**AT BE DE FR GB IT NL SE**

(56) References cited:
**EP-A-0 022 038**
**DE-C- 877 598**
**GB-A-2 048 267**
**US-A-3 248 432**
**US-A-4 168 391**
**US-A-4 233 466**

**CHEMISTRY LETTERS, vol. 1, no. 1, January 1981, TOKYO (JP), M. HIDAI et al.: "Homologation of methanol catalyzed by *mixed transition metal clusters containing* cobalt", pages 143-146**

(73) Proprietor: **TEXACO DEVELOPMENT CORPORATION**
**2000 Westchester Avenue**
**White Plains New York 10650 (US)**

(72) Inventor: **Knifton, John Frederick**
**10900 Catskill Trail**
**Austin Texas 78750 (US)**
Inventor: **Lin, Jiang-jen**
**2617 Oak Meadows Drive**
**Round Rock Texas 78664 (US)**

(74) Representative: **Brock, Peter William et al**
***Michael Burnside & Partners 2 Serjeants' Inn***
***Fleet Street***
**London EC4Y 1HL (GB)**

**EP 0 056 679 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to an improved process for preparing ethanol from methanol by reaction with hydrogen and carbon monoxide.

A great number of processes have been described in the art for reacting methanol with carbon monoxide and hydrogen in the presence of catalyst systems to produce ethanol. A general disadvantage of the art described processes is that they all produce a wide variety of other related products such as higher molecular weight alcohols, aldehydes, ketones, carboxylic acids, esters, etc. in addition to the desired ethanol.

In U.S. Patent No. 3,285,948, for example, a method of forming alcohols is set out in which a cobalt catalyst system comprising cobalt carbonyl, an iodine promoter and a ruthenium halide is described. Cawse discloses in U.S. Patent No. 4,013,700 a process for preparing polyhydric alcohols, etc. by reacting hydrogen and carbon monoxide in the presence of a quaternary phosphonium salt and a rhodium carbonyl at elevated temperature and pressure. Riley et al teach in U.S. Patent No. 3,248,432 the preparation of ethanol by the reaction of methanol, carbon monoxide, and hydrogen in the presence of a cobalt compound and an iodine promoter. Likewise in British Patent No. 1,546,428 the preparation of ethanol by reacting methanol with carbon monoxide and hydrogen in the presence of a solvent such as hydrocarbon solvent, a cobalt-containing catalyst such as cobalt iodide or bromide and a tertiary phosphine. Slinkard in U.S. Patent No. 4,168,391 teaches a process for preparing ethanol by reaction of carbon monoxide, hydrogen and methanol in the presence of cobalt carbonyl and an oxygenated solvent such as dioxane.

All of the processes described above suffer from one or more disadvantages. In most cases the conversion of methanol is low and a wide variety of products in addition to the desired ethanol are formed with consequent separation and disposal problems.

EP—A—0022038 discloses a liquid phase process for the hydrocarbonylation of methanol to ethanol, at a temperature of at least 180°C and a pressure between 50 and 400 bars, in the presence of cobalt and ruthenium. The reaction is carried out in the presence of an ionic halide, the cation being an alkali metal or alkaline earth metal or quaternary ammonium or phosphonium. It is essential that at least one alkyl halide is present and that the amounts of the various catalyst components are chosen to provide certain specific molar ratios, i.e.:

$X^-$: Co of at least 5 ($X^-$ being provided by the ionic halide);
X: Co of at least 2 (X being provided by the alkyl halide), and
Ru: Co of greater than 2.

In the process of this invention ethanol is prepared in high yield by reacting methanol with a mixture of hydrogen and carbon monoxide. More particularly, this invention relates to a process for preparing ethanol by contacting a mixture of carbon monoxide, hydrogen and methanol with a catalyst system characterized in that the catalyst comprises

(a) a ruthenium compound,
(b) a quaternary phosphonium or ammonium base or salt, and
(c) cobalt(II) iodide, cobalt(II) bromide or cobalt(II) chloride,

said contacting being carried out at a pressure of at least 35 bars and a temperature of at least 150°C.

According to one embodiment, the reaction can be carried out in the presence of an oxygenated hydrocarbon solvent.

Recovery of ethanol from the reaction product can be carried out in any conventional or convenient manner such as by distillation, extraction, etc.

The catalyst systems suitable for the practice of this invention comprise a ruthenium compound, a quaternary phosphonium or ammonium base or salt and a cobalt compound as exemplified by cobalt iodide. These catalyst systems give substantially higher yields of ethanol than can be obtained when the catalyst utilized is solely a ruthenium compound together with the quaternary base or salt. Likewise, when the catalyst system employed comprises, for example, only cobalt iodide and a tetraalkyl phosphonium salt, such as tetrabutylphosphonium bromide, no ethanol is formed. Furthermore, a high degree of conversion of methanol to the desired ethanol is achieved in this process. Also, the stability of this catalyst system is such that it can be conveniently recovered from the reaction mixture and recycled to the process.

Generally, with regard to the metallic components of the catalyst system it will contain from about 20 to about 80 mole percent of the ruthenium compound with the balance being cobalt halide based on the total number of moles of the ruthenium compound and the total number of moles of the cobalt compound in the system. Preferably, the catalyst system will contain about equimolar amounts of the ruthenium and cobalt compounds.

A wide variety of ruthenium compounds may be utilized in the catalyst system of this invention. For instance, the ruthenium may be added to the reaction mixture in an oxide form, as in the case of, for example, ruthenium(IV) oxide, hydrate, anhydrous ruthenium(IV) dioxide and ruthenium(VIII) tetraoxide. Alternatively, it may be added as the salt of a mineral acid, as in the case of ruthenium(III) chloride hydrate,

ruthenium(III) bromide, anhydrous ruthenium(III) chloride and ruthenium nitrate, or as the salt of a suitable organic carboxylic acid, for example, ruthenium(III) acetate, ruthenium(III) propionate, ruthenium butyrate, ruthenium(III) trifluoroacetate, ruthenium octanoate, ruthenium napththenate, ruthenium valerate and ruthenium(III) acetylacetonate. The ruthenium may also be added to the reaction zone as a carbonyl or hydrocarbonyl derivative. Here, suitable examples include triruthenium dodecacarbonyl, hydrocarbonyls such as $H_2Ru_4(CO)_{13}$ and $H_4Ru_4(CO)_{12}$, and substituted carbonyl species such as the tricarbonylruthenium(II) chloride dimer, $[Ru(CO)_3Cl_2]_2$.

Cobalt compounds suitable for use in this ruthenium-cobalt bimetallic catalyst system are cobalt(II) iodide, cobalt(II) bromide and cobalt(II) chloride. If desired, the cobalt(II) iodide can be generated in situ by adding the cobmination of cobalt and elemental iodide or hydrogen iodide to the reactor.

Quaternary phosphonium salts suitable for use in this process have the formula

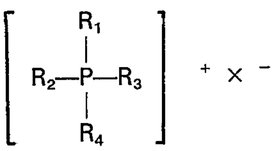

where $R_1$, $R_2$, $R_3$ and $R_4$ are organic radicals, particularly alkyl, aryl or alkaryl radicals bonded to the phosphorus atom, and X is an anionic species. The organic radicals useful in this instance include those alkyl radicals having 1 to 20 carbon atoms in a branched or linear alkyl chain; they include, for example, the methyl, ethyl, *n*-butyl, *iso*-butyl, octyl, 2-ethylhexyl and dodecyl radicals. Tetraoctylphosphonium bromide and tetrabutylphosphonium bromide are typical examples presently in commercial production. The corresponding quaternary phosphonium and ammonium acetates, hydroxides, nitrates, chromates, tetrafluoroborates and other halides, such as the corresponding chlorides, and iodides, are also satisfactory in this instance. Also useful are the corresponding quaternary ammonium bases and salts of the above series of compounds.

Equally useful are the phosphonium and ammonium salts containing phosphorus or nitrogen bonded to a mixture of alkyl, aryl and alkaryl radicals. Said aryl and alkaryl radicals may each contain 6 to 20 carbon atoms. The aryl radical is most commonly phenyl. The alkaryl group may comprise phenyl substituted with one or more $C_1$—$C_{10}$ alkyl substituents, bonded to the phosphorus or nitrogen atom through the aryl function.

Illustrative examples of suitable quaternary phosphonium and ammonium bases and salts include tetrabutylphosphonium bromide, tetraoctylphosphonium bromide, heptyltriphenylphosphonium bromide, tetrabutylphosphonium iodide, tetrabutylphosphonium chloride, tetrabutylphosphonium nitrate, tetrabutylphosphonium hydroxide, tetrabutylphosphonium chromate, tetrabutylphosphonium tetrafluoroborate, tetrabutylphosphonium acetate, tetrabutylammonium bromide and tetramethylammonium hydroxide, pentahydrate and trimethyldodecylammonium bromide.

The preferred quaternary salts are generally the tetraalkylphosphonium or alkyl-triaryl salts containing alkyl groups having 3—8 carbon atoms, such as butyl, hexyl and octyl and where the aryl group is phenyl. Tetrabutylphosphonium salts, such as tetrabutylphosphonium bromide, constitute a preferred group of tetraalkylphosphonium salts for the practice of this invention.

Preferred tetrabutylphosphonium salts or bases include the bromide, chloride, iodide, acetate, the chrome salts and hydroxide base. Preferred alkyl-triaryl phosphonium salts include, for example, heptyltriphenylphosphonium bromide, butyltriphenylphosphonium bromide, and methyltriphenylphosphonium bromide as well as the corresponding chlorides.

Generally, in the catalyst system the molar ratio of the ruthenium compound to the quaternary phosphonium or ammonium salt or base will range from about 1:0.01 to about 1:100 or more and, preferably, will be from about 1:0.5 to about 1:20.

The quantity of ruthenium compound employed in the instant invention is not critical and may vary over a wide range. In general, the novel process is desirably conducted in the presence of a catalytically effective quantity of the active ruthenium species and of the cobalt iodide which gives the desired product in reasonable yield. The reaction proceeds when employing as little as about $1 \times 10^{-6}$ weight percent, and even lesser amounts of ruthenium together with about $1 \times 10^{-6}$ wight percent or less of cobalt, basis the total weight of the reaction mixture. The upper concentration is dictated by a variety of factors including catalyst cost, partial pressures of carbon monoxide and hydrogen, operating temperature etc. A ruthenium concentration of from about $1 \times 10^{-5}$ to about 5 weight percent in conjunction with a cobalt concentration of from about $1 \times 10^{-5}$ to about 5 weight percent, based on the total weight of reaction mixture is generally desirable in the practice of this invention.

The solvent useful in the process of this invention is an oxygenated hydrocarbon i.e., a compound composed only of carbon, hydrogen and oxygen and one in which the only oxygen atoms present are in ether groups, ester groups, ketone carbonyl groups or hydroxyl groups of alcohols. Generally, the oxygenated hydrocarbon will contain 3 to 12 carbon atoms and preferably a maximum of 3 oxygen atoms. The solvent must be substantially inert under reaction conditions, it must be relatively non-polar and it must be one which has a normal boiling point of at least 65°C at atmospheric pressure and preferably, the

solvent will have a boiling point greater than that of ethanol and other oxygen-containing reaction products so that recovery of the solvent by distillation is facilitated.

Preferred ester type solvents are the aliphatic and acrylic carboxylic acid monoesters as exemplified by butyl acetate, methyl benzoate, isopropyl iso-butyrate, and propyl propionate as well as dimethyl adipate. Useful alcohol-type solvents include monohydric alcohols such as cyclohexanol, 1-hexanol, 2-hexanol, neopentanol, 2-octanol, etc. Suitable ketone-type solvents include, for example, cyclic ketones such as cyclohexanone, 2-methylcyclohexanone, as well as acyclic ketones such as 2-pentanone, butanone, acetophenone, etc. Ethers which may be utilized as solvents include cyclic, acyclic and heterocyclic materials. Preferred ethers are the heterocyclic ethers as illustrated by 1,4-dioxane and 1,3-dioxane. Other suitable ether solvents include isopropyl propyl ether, diethylene glycol dibutyl ether, dibutyl ether, ethyl butyl ether, diphenyl ether, heptyl phenyl ether, anisole, tetrahydrofuran, etc. The most useful solvents of all of the above groups include the ethers as represented by monocyclic, heterocyclic ethers such as 1,4-dioxane, etc.

The temperature range which can usefully be employed in these syntheses is a variable dependent upon other experimental factors, including the pressure, and the concentration and choice of a particular species of ruthenium catalyst among other things. The range of operability is from 150° to 350°C when superatmospheric pressure of syngas (i.e. a mixture of carbon monoxide and hydrogen produced by the partial oxidation of a hydrocarbonaceous fuel), are employed. A narrow range of 180—250°C represents the preferred temperature range.

Superatmospheric pressures of at least 35 bars lead to substantial yields of ethanol by the process of this invention. A preferred operating range is from 135 to 700 bars, although pressures above 700 bars also provide useful yields of ethanol.

The relative amounts of carbon monoxide and hydrogen which may be initially present in the syngas mixture can be varied widely. In general, the mole ratio of Co to $H_2$ is in the range from about 20:1 up to about 1:20, preferable from about 5:1 to 1:5, although ratios outside these ranges may also be employed. Particularly in continuous operations, but also in batch experiments, the carbon monoxide-hydrogen gaseous mixtures may also be used in conjunction with up to 50% by volume of one or more other gases. These other gases may include one or more inert gases such as nitrogen, argon, neon and the like, or they may include gases that may, or may not, undergo reaction under CO hydrogenation conditions, such as carbon dioxide, hydrocarbons such as methane, ethane, propane and the like, ethers such as dimethyl ether, methylethyl ether and diethyl ether, alkanols such as methanol and acid esters such as methyl acetate.

Higher alcohols and carboxylic acid esters may also be formed while carrying out the process of this invention. Most often these derivatives are n-propanol, methyl formate, methyl acetate, ethyl acetate, ethyl ether, etc. The major by-products of the process such as the higher molecular weight alcohols and carboxylic acid esters, are, of course, also useful compounds and major articles of commerce. The higher alcohols, the carboxylic acid esters and ethers can easily be separated from one another by conventional means, e.g., fractional distillation in vacuo.

The novel process of this invention can be conducted in a batch, semi-continuous or continuous fashion. The catalyst may be initially introduced into the reaction zone batchwise, or it may be continuously or intermittently introduced into such a zone during the course of the synthesis reaction. Operating conditions can be adjusted to optimize the formation of the ethanol product, and after recovery of the alcohol and other products, a fraction rich in ruthenium catalyst components may then be recycled to the reaction zone, if desired, and additional products generated.

The products have been identified in this work by one or more of the following analytical procedures, viz., gas-liquid phase chromatograph (GLC), infrared (IR), mass spectrometry, nuclear magnetic resonance (nmr) and elemental analyses, or a combination of these techniques. Analyses have, for the most part, been by parts in weight; all temperatures are in degrees centigrade and all pressures in bars.

The following examples illustrate the novel process of this invention.

Example 1

A glass reactor liner was charged with 0.57 g (3.0 mmoles) of hydrated ruthenium(IV) dioxide, 10.2 g (30 mmoles) of n-tetrabutylphosphonium bromide, 1.9 g (6.0 mmoles) of cobalt(II) iodide, 30 ml of methanol and 70 ml of p-dioxane. The glass liner was placed in a stainless steel reactor. The reactor was purged of air and pressured to 70 bars with a mixture of carbon monoxide and hydrogen (1:2 molar) then was heated to 200°C, while it was agitated by rocking. The pressure was brought up to 235.5 bars and constant pressure was maintained by repressuring from a surge tank.

The reaction was stopped after 10 hours and the reactor cooled to room temperature. An off-gas sample was taken and excess gas vented from the reactor following which 115.8 g of a reddish-brown product was recovered.

Analysis of the liquid product by GLC showed the following product composition:

74 mole % ethanol
4 mole % n-propanol
0 mole % methyl formate

1 mole % ethyl acetate
0 mole % ethyl ether.

The methanol conversion was calculated to be 80 mole percent. The water content as determined by Karl Fischer titration was 2.06 mole percent.

A typical off-gas sample showed the presence of:

33.8% hydrogen
8.2% carbon monoxide
40.0% carbon dioxide
0.5% ethane.

Example 2

In this comparative example the experimental procedure of Example 1 was followed. The reactor was charged with 0.31 g (1.0 mmole) of cobalt(II) iodide, 1.7 g (5.0 mmoles) of tetrabutylphosphonium bromide, 8 ml of methanol and 20 ml of p-dioxane. No ruthenium was present in this run. After pressuring to 70 bars with a mixture of carbon monoxide and hydrogen (1:2 molar), the reactor was heated to 200°C; while it was agitated by rocking. The pressure was brought up to 270 bars and constant pressure was maintained by repressuring from a surge tank. After 18 hours, the reactor was cooled rapidly and the residual pressure (162 bars) was noted. Excess gas was removed by depressuring and a reddish-brown liquid product (29.9 g) recovered from the glass reactor liner.

Analysis of the liquid product by GLC showed the presence of 47 mole percent of ethyl acetate and no ethanol was detected. The methanol conversion was 30 mole percent.

Example 3

The reactor was charged with 0.10 g (0.5 mmole) of ruthenium(IV) dioxide hydrate, 1.70 g (5.0 mmoles) of n-tetrabutylphosphonium bromide, 8 ml of methanol and 20 ml of p-dioxane. No cobalt(II) iodide was present in this run. The reactor was pressured to 139 bars with a mixture of carbon monoxide and hydrogen (1:2 molar) and then heated to a temperature of 200°C while it was agitated by rocking. The pressure was brought up to 442 bars and these conditions of temperature and pressure held for 18 hrs. No surge tank was used and the pressure dropped to 373 bars during the reaction process.

At the end of 18 hours the reactor was cooled, an off-gas sample was taken and the excess gas released. The reddish-brown liquid product recovered (28.8 g) was analyzed by GLC to give 61 mole percent ethanol product selectivity. Methanol conversion was 11 percent.

This experiment showed the very low conversion of methanol to ethanol when the catalyst system does not contain cobalt iodide.

Example 4

Following the general procedure of Examples 1—3 inclusive, a glass liner reactor was charged with 0.57 g (3.0 mmoles) of hydrated ruthenium oxide, 10.2 g (30 mmoles) tetra-n-butylphosphonium bromide, 1.9 g (6 mmoles) of cobalt(II) iodide, 30 ml of methanol and 70 ml of p-dioxane. After flushing with syngas ($CO/H_2$ mixture), the reactor was pressured to 70 bars with a gaseous mixture containing 2 moles of hydrogen per mole of carbon monoxide, and heated to 200°C with agitation. Then the pressure was brought up to 235.5 bars and these conditions were maintained for 15 hours. After the indicated reaction time, the reactor was cooled and vented and the reddish-brown product recovered and analyzed by GLC and Karl Fischer Titration. There were no residual solids at this stage.

The product liquid was distilled at atmospheric pressure and a distillate fraction was collected at bp range of ca. 60—100°C. The residual catalyst remained behind as a deep-brown colored liquid (ca. 9.5 g). An aliquot of residual catalyst liquid (ca. 4.5 g), 8 ml of methanol and 24 ml of p-dioxane was charged to the glass liner reactor. The reactor was sealed, flushed with syngas, pressured to 70 bars with $CO/H_2$ (1:2) and heated to 200°C with agitation. The pressure was brought up to 346 bars and maintained for 18 hours. In this manner the synthesis of ethanol was repeated successfully, and the latter recovered from the crude liquid product by simple distillation.

The residual catalyst solution (5.0 g) from this second cycle was again returned to the reactor for further ethanol synthesis. The syngas pressures of 288 bars and 260 bars were used for the third and fourth cycles respectively. The methanol conversion and ethanol selectivity for this four cycle experiment are shown in Table 1.

TABLE 1
Synthesis of ethanol from methanol and syngas—
catalyst recycling

| Example | Number of catalyst cycles | Methanol conversion | Ethanol selectivity |
|---|---|---|---|
| 4 | 1 | 80 | 74 |
| | 2 | 88 | 73 |
| | 3 | 86 | 70 |
| | 4 | 69 | 77 |

Examples 5—7

Using the procedures and ruthenium-cobalt catalyst of Example 1, methanol homologation to ethanol was conducted at 200°C, and 235.5 bars operating pressure for various reaction periods. Table 2 summarizes the results for methanol conversion and ethanol selectivity.

TABLE 2

| Example | Time (hr.) | Ru/Co Molar ratio | MeOH Conversion | EtOH Selectivity |
|---|---|---|---|---|
| 5 | 1 | 1:2 | 22 | 62 |
| 6 | 5 | 1:2 | 44 | 83 |
| 7 | 9 | 1:2 | 70 | 79 |

Example 8

A glass liner reactor was charged with 0.38 g (2.0 mmoles) of hydrated ruthenium oxide, 0.63 g (2.0 mmoles) of cobalt(II) iodide, 3.53 g (8 mmoles) of n-heptyltriphenylphosphonium bromide and 20 ml of methanol. The glass liner was placed in a stainless steel reactor, the reactor was purged of air and pressured to 104 bars with a mixture of carbon monoxide and hydrogen (1:1 molar) and then heated to 200°C while it was agitated by rocking. The pressure was brought up to 277 bars and held at 200°C for 18 hours. As the reaction proceeded the pressure dropped to 223 bars.

The reaction was stopped after 18 hours and the reactor cooled to room temperature (final pressure—124.5 bars. An off-gas sample was collected and excess gas was vented after which 24.1 g of a reddish-brown product was collected.

Analysis of the liquid product by GLC showed the following product yield composition:

64 mole % ethanol
5 mole % n-propanol
9 mole % methyl formate
8 mole % ethyl acetate
3 mole % ethyl ether.

The methanol conversion was calculated to be 59 mole %.

A typical off-gas sample showed the presence of:

29.1% hydrogen
5.6% carbon monoxide
9.3% methane
49.3% carbon dioxide
0.7% ethanol.

Examples 9 and 10

Following the general procedure of Example 8, two additional catalyst systems were employed in preparing ethanol from methanol by the process of this invention. Details relating to the catalyst composition and other conditions are set out in Table 3 which follows.

The data in these examples indicate that a high degree of methanol conversion and a high degree of selectivity to ethanol in the liquid product was achieved.

TABLE 3

Homologation of methanol

| Example | Catalyst composition | MeOH Conversion (mole %) | Selectivity (mole %) | | | | | |
|---|---|---|---|---|---|---|---|---|
| | | | EtOH | n-PrOH | MeOOCH | MeOAc | EtOAc | $Et_2O$ |
| $9_a$ | $RuO_2$-n-$C_7H_{15}Ph_3PBr$-$CoI_2$ | 61 | 68 | 5 | 18 | 12 | 5 | 5 |
| $10_b$ | $RuO_2$-n-$Bu_4PBr$-$CoI_2$ | 80 | 56 | 4 | 5 | 10 | 12 | 2 |

[a] Run Conditions: 0.38 g (2.0 mmoles) $RuO_2$; 3.53 g (8 mmoles) n-$C_7H_{15}Ph_3PBr$; 1.26 g (4.0 mmoles) $CoI_2$; MeOH 20 ml; time 18 hours; temp. 200°C, initial pressure 277 bars; $H_2/CO$ (1:1 molar).

[b] Run Conditions: 0.38 g (2.0 mmoles) $RuO_2$; 6.8 g (20 mmoles) n-$Bu_4PBr$; 1.26 g (4.0 mmoles) $CoI_2$; MeOH 20 ml; time 18 hours; temp. 200°C; initial pressure 277 bars $H_2/CO$ (1:1 molar).

Example 11

In this comparative example the experimental procedure of Example 8 was followed. The reactor was charged with 0.38 g (2.0 mmole) of hydrated ruthenium(IV) dioxide, 3.53 g (8.0 mmoles) of n-heptyltriphenylphosphonium bromide and 20 ml of methanol. No cobalt(II) iodide was present in this run. After pressuring to 104.5 bars with a mixture of carbon monoxide and hydrogen (1:1 molar), the reactor was heated to 200°C while it was agitated by rocking. The pressure was then raised to 277 bars with the same carbon monoxide-hydrogen mixture and maintained at 200°C for 18 hours, as the reaction proceeded the pressure dropped to 256 bars. The reactor was cooled rapidly and the residual pressure (145 bars) was noted. Excess gas was removed by depressuring and a reddish-brown liquid product 21.7 g recovered from the glass reactor liner.

Analysis of the liquid product by GLC showed the following product yield composition:

30 mole % ethanol
2 mole % n-propanol
14 mole % methyl formate
7 mole % methyl acetate.

The methanol conversion was 26 mole percent.

The results of this experiment show the low yield of ethanol as well as the low conversion of methanol when cobalt(II) iodide is omitted from the catalyst system.

Example 12

In this comparative example the experimental procedure of Example 8 was followed. The reactor was charged with 0.38 g (2.0 mmoles) of hydrated ruthenium dioxide, 0.25 g (1.0 mmole) of iodine and 15 ml of methanol. No quaternary phosphonium or ammonium base or salt and no cobalt(II) iodide was present in this run. Using a 1:1 (molar) mixture of carbon monoxide and hydrogen the reactor was pressured to 70 bars and heated to a temperature of 200°C while it was agitated by rocking. Next the reactor was pressured to 277 bars using the same carbon monoxide-hydrogen mixture and held at 200°C for 18 hours. At the end of the reaction period the pressure was 244 bars.

The reactor was cooled to room temperature, an off-gas sample was taken and the excess gas released. The reddish-brown liquid product which was recovered (9.1 g) was analyzed by GLC, product composition was as follows:

75 mole % ethanol
2 mole % n-propanol
9 mole % methyl formate
2 mole % methyl acetate
1 mole % ethyl ether.

The methanol conversion was 26 mole percent.

The data in this experiment show the very low conversion of methanol achieved when no quaternary salt or cobalt(II) iodide is present in the catalyst system.

Example 13

The experimental procedure of Example 8 was employed in this comparative example. The reactor was charged with 0.38 g (2.0 mmoles) of hydrated ruthenium dioxide, 3.53 g (8.0 mmoles) of n-heptyltriphenylphosphonium bromide, 0.25 g (1.0 mmole) of iodine and 15 ml of methanol. No cobalt(II) iodide was present in this example. The reactor was pressured to 70 bars using a 1:1 molar mixture of carbon monoxide and hydrogen and heated to a temperature of 200°C while it was agitated by rocking. Using the same carbon monoxide-hydrogen mixture the reactor was pressured to 277 bars and maintained at 200°C for 18 hours. At the end of the reaction period the pressure was 257.5 bars.

The reactor was cooled to room temperature, an off-gas sample was taken and excess gas was vented. A reddish-brown liquid product (16.2 g) which was recovered was analyzed by GLC showed the following product composition:

52 mole % ethanol
7 mole % n-propanol
15 mole % methyl formate
7 mole % methyl acetate
1 mole % ethyl ether.

Methanol conversion was 19 mole percent. These results show that a very low degree of methanol conversion resulted when cobalt(II) iodide was not present in the reaction mixture.

**Claims**

1. A process for preparing ethanol by contacting a mixture of carbon monoxide, hydrogen and methanol with a catalyst system characterized in that the catalyst comprises

(a) a ruthenium compound,
(b) a quaternary phosphonium or ammonium base or salt, and
(c) cobalt(II) iodide, cobalt(II) bromide or cobalt(II) chloride,

said contacting being carried out at a pressure of at least 35 bars and a temperature of at least 150°C.

2. A process according to Claim 1 characterized in that contacting is carried out in the presence of an inert liquid oxygenated hydrocarbon solvent.

3. A process according to Claim 2 characterized in that the solvent is 1,3-dioxane, 1,4-dioxane, isopropyl propyl ether, diethylene glycol dibutyl ether, dibutyl ether or ethyl butyl ether.

4. A process according to any preceding Claim characterized in that the pressure is from 135 to 700 bars.

5. A process according to any preceding Claim characterized in that the temperature is from 180 to 250°C.

6. A process according to any preceding Claim characterized in that the ruthenium compound is anhydrous ruthenium(IV) dioxide, ruthenium(IV) dioxide hydrate, ruthenium(VIII) tetraoxide, ruthenium(III) trichloride, ruthenium(III) acetate, ruthenium(III) propionate, ruthenium(III) acetylacetonate or triruthenium dodecacarbonyl.

7. A process according to any preceding Claim characterized in that the quaternary compound (b) is a tetraalkylphosphonium salt or base.

8. A process according to Claim 7 characterized in that the tetraalkylphosphonium salt or base is tetrabutyl-phosphonium chloride, bromide, iodide, acetate, chromate or hydroxide.

9. A process according to any of Claims 1 to 7 characterized in that the quaternary compound (b) is an alkyl-triarylphosphonium salt.

10. A process according to Claim 9 characterized in that the alkyl-triarylphosphonium salt is heptyltriphenylphosphonium bromide, heptyltriphenylphosphonium chloride, or methyltriphenylphosphonium bromide.

**Patentansprüche**

1. Verfahren zur Herstellung von Ethanol durch Umsetzung eines Gemisches von Kohlenmonoxid, Wasserstoff und Methanol mit einem Katalysatorsystem, dadurch gekennzeichnet, daß der Katalysator die folgenden Bestandteile enthält oder daraus besteht:

(a) eine Ruthenium-Verbindung,
(b) eine quarternäre Phosphonium- oder Ammonium-Base oder Salz, und
(c) Cobalt(II)jodid, Cobalt(II)bromid oder Cobalt(II)chlorid,

wobei die Umsetzung bei einem Druck von mindestens 25 bar und einer Temperatur von mindestens 150°C erfolgt.

2. Verfahren nach Anspruch 1, dadurch gekennzeichnet, daß die Umsetzung in Gegenwart eines inerten flüssigen oxygenierten Kohlenwasserstoff-Lösungsmittels erfolgt.

3. Verfahren nach Anspruch 2, dadurch gekennzeichnet, daß das Lösungsmittel 1,3-Dioxan, 1,4-Dioxan, Isopropylpropylether, Diethylenglykol-dibutylether, Dibutylether oder Ethylbutylether ist.

4. Verfahren nach einem der vorhergehenden Ansprüche, dadurch gekennzeichnet, daß der Druck 135 bis 700 bar beträgt.

5. Verfahren nach einem der vorhergehenden Ansprüche, dadurch gekennzeichnet, daß die Temperatur 180 bis 250°C beträgt.

6. Verfahren nach einem der vorhergehenden Ansprüche, dadurch gekennzeichnet, daß die Ruthenium-Verbindung wasserfreies Ruthenium(IV)dioxid, Ruthenium(IV)dioxid-Hydrat, Ruthenium(VIII)tetraoxid, Ruthenium(III)trichlorid, Ruthenium(III)acetat, Ruthenium(III)propionat, Ruthenium(III)acetylacetonat oder Triruthenium-dodecacarbonyl ist.

7. Verfahren nach einem der vorhergehenden Ansprüche, dadurch gekennzeichnet, daß die quarternäre Verbindung (b) ein Tetraalkylphosphonium-Salz oder Base ist.

8. Verfahren nach Anspruch 7, dadurch gekennzeichnet, daß das Tetraalkylphosphonium-Salz oder Base Tetrabutylphosphonium-chlorid, -bromid, -jodid, -acetat, -chromat oder -hydroxid ist.

9. Verfahren nach einem der Ansprüche 1 bis 7, dadurch gekennzeichnet, daß die quarternäre Verbindung (b) ein Alkyltriarylphosphonium-Salz ist.

10. Verfahren nach Anspruch 9, dadurch gekennzeichnet, daß das Alkyltriarylphosphonium-Salz Heptyltriphenylphosphonium-bromid, Heptyltriphenylphosphonium-chlorid oder Methyltriphenylphosphonium-bromid ist.

**Revendications**

1. Procédé de préparation d'éthanol par mise en contact d'un mélange de monoxyde de carbone, d'hydrogène et de méthanol avec un système catalytique, caractérisé en ce que le catalyseur comprend:

(a) un composé de ruthénium,
(b) un sel ou une base de phosphonium ou d'ammonium quaternaire, et
(c) de l'iodure de cobalt(II), du bromure de cobalt(II) ou du chlorure de cobalt(II),

cette mise en contact étant réalisée à une pression d'au moins 35 bars et à une température d'au moins 150°C.

2. Procédé suivant la revendication 1, caractérisé en ce que la mise en contact est réalisée en présence d'un solvant hydrocarburé oxygéné liquide inerte.

3. Procédé suivant la revendication 2, caractérisé en ce que le solvant est le 1,3-dioxane, le 1,4-dioxane, l'éther isopropyl propylique, l'éther dibutylique de diéthylène glycol, l'éther dibutylique ou l'éther éthyl butylique.

4. Procédé suivant l'une quelconque des revendications précédentes, caractérisé en ce que la pression est de 135 à 700 bars.

5. Procédé suivant l'une quelconque des revendications précédentes, caractérisé en ce que la température est de 180 à 250°C.

6. Procédé suivant l'une quelconque des revendications précédentes, caractérisé en ce que le composé de ruthénium est le dioxyde de ruthénium(IV) anhydre, le dioxyde de ruthénium(IV) hydraté, le tétroxyde de ruthénium(VIII), le trichlorure de ruthénium(III), l'acétate de ruthénium(III), le propionate de ruthénium(III), l'acétylacétonate de ruthénium(III) ou le triruthénium dodécacarbonyle.

7. Procédé suivant l'une quelconque des revendications précédentes, caractérisé en ce que le composé quaternaire (b) est un sel ou une base de tétraalkylphosphonium.

8. Procédé suivant la revendication 7, caractérisé en ce que le sel ou la base de tétraalkylphosphonium est du chlorure, du bromure, de l'iodure, de l'acétate, du bromate ou de l'hydroxyde de tétrabutylphosphonium.

9. Procédé suivant l'une quelconque des revendications 1 à 7, caractérisé en ce que le composé quaternaire (b) est un sel d'alkyltriarylphosphonium.

10. Procédé suivant la revendication 9, caractérisé en ce que le sel d'alkyltriarylphosphonium est le bromure d'heptyltriphénylphosphonium, le chlorure d'heptyltriphénylphosphonium ou le bromure de méthyltriphénylphosphonium.